# FASCICULE DE BREVET EUROPEEN

(11) **EP 1 819 680 B1**
(45) Date de publication et mention de la délivrance du brevet: **15.02.2012**
(21) Numéro de dépôt: 05822938.6
(22) Date de dépôt: 25.11.2005
(51) Int. Cl.: C07D 223/12, A61K 31/55, C07D 403/06

(54) **BENGAMIDES POSSEDANT UN CYCLE CAPROLACTAME SUBSTITUE, PROCEDE DE PREPARATION, COMPOSITIONS LES CONTENANT ET UTILISATION**
BENGAMIDE MIT EINEM SUBSTITUIERTEN CAPROLACTAM-ZYKLUS, VERFAHREN ZU IHRER HERSTELLUNG, ZUSAMMENSETZUNGEN MIT IHNEN UND IHRE VERWENDUNG
BENGAMIDES WITH A SUBSTITUTED CAPROLACTAME CYCLE, METHOD FOR THE PREPARATION THEREOF, COMPOSITIONS CONTAINING THEM AND USE THEREOF

(30) Priorité: 29.11.2004 FR 0412646
(43) Date de publication de la demande: 22.08.2007
(73) Titulaire: Aventis Pharma S.A., 92160 Antony (FR)
(72) Inventeur: ZHANG, Jidong, F-75013 Paris (FR); BHATNAGAR, Neerja, Neshanic Station, New Jersey 08853 (US)
(74) Mandataire: Michiels, Stéphanie Laure
(86) Numéro de dépôt international: PCT/FR2005/002931
(87) Numéro de publication internationale: WO 2006/056695

(56) Documents cités:
- WO-A-00/29382
- WO-A-2005/014574
- WO-A-2005/044803
- KINDER F R ET AL: "SYNTHESIS AND ANTITUMOR ACTIVITY OF ESTER-MODIFIED ANALOGUES OF BENGAMIDE B" JOURNAL OF MEDICINAL CHEMISTRY, AMERICAN CHEMICAL SOCIETY. WASHINGTON, US, vol. 44, 2001, pages 3692-3699, XP002310388 ISSN: 0022-2623 cité dans la demande
- PATENT ABSTRACTS OF JAPAN vol. 2003, no. 12, 5 décembre 2003 (2003-12-05) -& JP 2004 262793 A (NOYAKU BIO TECHNOLOGY KAIHATSU GIJUTSU KENKYU KUMIAI), 24 septembre 2004 (2004-09-24)

## Description

La présente invention concerne notamment des bengamides possédant un cycle caprolactame substitué, leur procédé de préparation, des compositions les contenant, et leur utilisation comme médicament.

Plus particulièrement, et selon un premier aspect, l'invention concerne des caprolactames substitués utiles comme agents anticancéreux.

Des bengamides ont été décrits dans US 6239127, US 20010044433 A1, WO 01185697, WO 00129382, US 4831135, EP 687673, et US 2002128474 A1. Ces documents divulguent essentiellement des analogues et dérivés de bengamide, un produit naturel isolé d'une éponge marine, *Jaspis coriacea.*

Ces mêmes produits ont été décrits dans la littérature : J. Org. Chem. (1986), 51(23), 4494-7 ; J. Org. Chem. (2001), 66(5), 1733-41 ; J. Med. Chem. 2001, 44, 3692-9.

Kinder et al, dans J. Med. Chem. 2001, 44, 3692-9, présentent l'activité de ces différents bengamides. Dans cette étude, les auteurs expliquant que la présence d'un ester lipophile sur le caprolactame est essentiel pour leur activité anticancéreuse *in vitro*, et que la N-substitution du lactame par un méthyle n'a pas d'effet sur l'activité précitée.

Contre toute attente, il a été trouvé qu'il est possible d'obtenir des produits ayant une activité anticancéreuse significative en modifiant les substituants portés par l'azote du caprolactame.

On peut notamment citer les produits de formule (I) suivante : 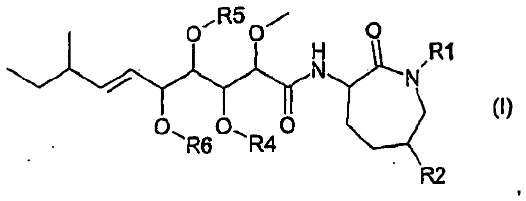 dans laquelle:
(i) R1 est indépendamment sélectionné dans le groupe constitué par H,-(C1-C24)alkyle, -(C3-C9)cycloalkyle, hétérocycloalkyle, -(C3-C24)alkylène, hétérocycloalkylène, aryle, hétéroaryle, arylalkyle, hétéroarylalkyle, arylalkylène, hétéroarylalkylène, -(C1-C8)alkyl-aryl-(C1-C24)alkyle, -(C1-C8)alkyl-aryl-O-(C1-C24)alkyle,
(ii) R2 est indépendamment sélectionné dans le groupe constitué par H, OR7, OCO(R7), dans lequel R7 est sélectionné dans le groupe constitué par -(C1-C24)alkyle, (C3-C9)cycloalkyle, hétérocycloalkyle, -(C3-C24)alkylène, hétérocycloalkylène, aryle, hétéroaryle, arylalkyle, hétéroarylalkyle, arylalkylène, hétéroarylalkylène, -(C1-C8)alkyl-aryl-(C1-C24)alkyle, -(C1-C8)alkyl-aryl-O-(C1-C24)alkyle,
(iii) R4, R5, et R6 sont chacun indépendamment sélectionnés dans le groupe constitué par H, -(C1-C6)acyle, -(C1-C6)alkyle, -(C1-C6)alkyl-aryle,-(C1-C6)alkyl-hétéroaryle, -aryle, -hétéroaryle, -arylalkylène,-hétéroarylalkylène,
sous réserve que lorsque R4, R5, R6 sont chacun H, et R1 est H ou méthyle, alors R2 n'est pas H ou OH.

Avantageusement, R4, R5, et R6 sont chacun indépendamment sélectionnés dans le groupe constitué par H et -(C1-C6)acyle.

R4, R5, et R6 sont de préférence H.

Un R2 préféré est choisi parmi H et OH, et R2 encore préféré est H.

La présente invention concerne, un produit de formule générale (II) suivante : 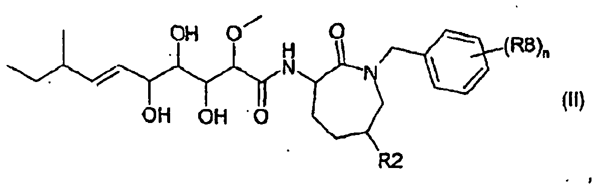 dans lequel R8 est sélectionné dans le groupe constitué par H, halogène, OH, CN, O(C1-C24)alkyle, OCO(C1-C24)alkyle, -(C1-C4)alkyl-aryle, -(C1-C4)alkyl-hétéroaryle; dans lequel n = 0, 1, 2, 3, 4 ou 5, et dans lequel R2 est sélectionné dans le groupe constitué par H et OH.

Les composés préférés de l'invention répondent à la formule (II) dans laquelle R8 est sélectionné parmi H et F, et dans laquelle n = 0, 1, 2, 3, 4, ou 5. Avantageusement, dans la formule (II), n = 4 ou 5.

De manière préférée, l'invention concerne les produits exemplifiés dans les tableaux 1 et 2. Ils peuvent exister à l'état de bases, de sels d'additions avec des acides, de solvats ou d'hydrates.

Les produits de formule générale (I) telle que défini ci-desus peuvent être préparés selon un procédé comprenant les étapes suivantes :
1) Mise en culture et croissance de *Myxococcus virescens*,
2) Extraction d'une fraction riche en bengamides de ladite culture,
3) Introduction des substituants R1 à R8 sur un produit issu de la fraction riche en bengamides, pour l'obtention d'un produit de formule générale (I).

Ce procédé comprend avantageusement une étape de purification de la fraction riche en bengamides préalablement à l'étape 3.

La fraction riche en bengamides comprend des produits de formule générale (IV) suivante: 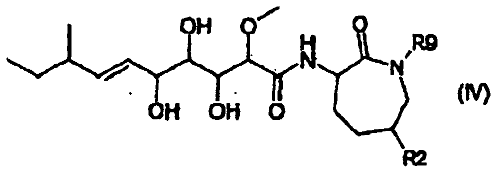 dans laquelle R9 est H ou méthyle, et R2 est H ou OH.

L'étape 3 d'introduction des substituants R1 à R6 comprend avantageusement une étape dans laquelle le substituant R1 est introduit sur le produit de formule générale (IV) après protection de ses fonctions alcool libres.

La présente invention concerne également un procédé de préparation d'un produit de formula générale (II) telle que définie ci-dessus 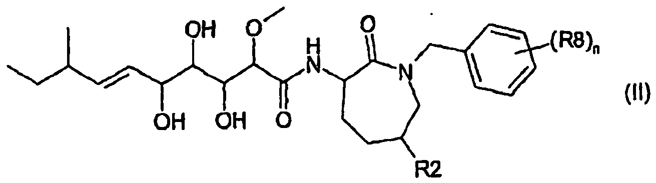 dans laquelle R2 est H ou OH, et R8 est telle que défini ci-dessus comprenant une étape dans laquelle un produit de formule générale (VI) suivante : 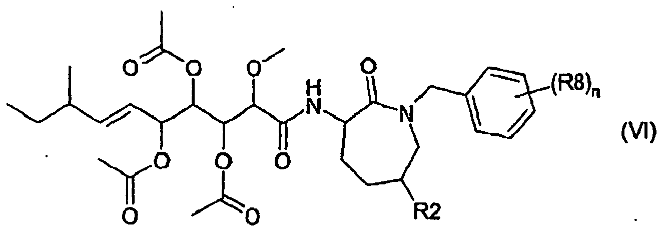 dans laquelle R2 est H ou OCOCH₃, et R8 et n sont tels que définis précédemment, est saponifié pour l'obtention d'un produit de formule générale (II).

Le produit de formule générale (VI) est avantageusement obtenu par réaction entre un produit de formule générale (V) suivante : 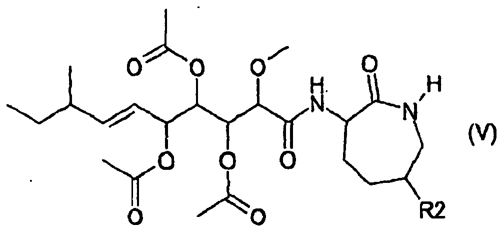 et un halogénure de benzyle 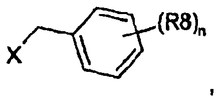 dans lequel X est un halogène et R8 et n sont tels que définis précédemment, en présence d'une base.

Le produit de formule générale (V) est avantageusement obtenu par acétylation d'un produit de formule générale (IV) suivante : 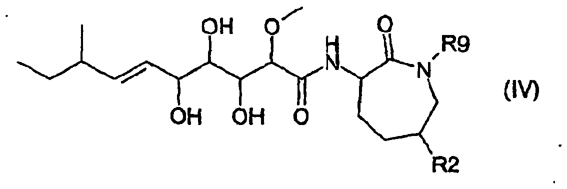 dans laquelle R9 est H, et R2 est H ou OH.

Selon un troisième aspect, l'invention concerne l'utilisation d'un produit selon son premier ou selon son second aspect pour la fabrication d'un médicament utile pour traiter un état pathologique, de préférence le cancer.

Les produits selon l'invention peuvent être sous forme non chirale, ou racémique, ou enrichie en un stéréo-isomère, ou enrichie en un énantiomére ; et peuvent éventuellement être saliflés.

Un produit conforme à l'invention pourra être utilisé pour la fabrication d'un médicament utile pour traiter un état pathologique, en particulier un cancer.

La présente invention concerne également un produit tel que défini ci-dessus pour son utilisation pour le traitement d'un état pathologique, notamment pour le traitement du cancer.

La présente invention concerne aussi les compositions thérapeutiques contenant un composé selon l'invention, en association avec un excipient pharmaceutiquement acceptable selon le mode d'administration choisi. La composition pharmaceutique peut se présenter sous forme solide, liquide ou de liposomes.

Parmi les compositions solides on peut dter les poudres, les gélules, les comprimés. Parmi les formes orales on peut aussi inclure les formes solides protégées vis-à-vis du milieu acide de l'estomac. Les supports utilisés pour les formes solides sont constitués notamment de supports minéraux comme les phosphates, les carbonates ou de supports organiques comme le lactose, les celluloses, l'amidon ou les polymères. Les formes liquides sont constituées de solutions de suspensions ou de dispersions. Elles contiennent comme support dispersif soit l'eau, soit un solvant organique (éthanol, NMP ou autres) ou de mélanges d'agents tensioactifs et de solvants ou d'agents complexants et de solvants.

Les formes liquides seront de préférence injectables et, de ce fait, auront une formulation acceptable pour une telle utilisation.

Des voies d'administration par injection acceptables incluent les voies intraveineuse, intra-péritonéale, intramusculaire, et sous cutanée, la voie intraveineuse étant préférée.

La dose administrée des composés de l'invention sera adaptée par le praticien en fonction de la voie d'administration du patient et de l'état de ce dernier.

Les composés de la présente invention peuvent être administrés seuls ou en mélange avec d'autres anticancéreux. Parmi les associations possibles on peut citer :
- les agents alkylants et notamment le cyclophosphamide, le melphalan, l'ifosfamide, le chlorambucil, le busulfan, le thiotepa, la prednimustine, la carmustine, la lomustine, la semustine, la steptozotocine, la decarbazine, la témozolomide, la procarbazine et l'hexaméthylmélamine
- les dérivés du platine comme notamment le cisplatine, le carboplatine ou l'oxaliplatine
- les agents antibiotiques comme notamment la bléomycine, la mitomycine, la dactinomycine
- les agents antimicrotubules comme notamment la vinblastine, la vincristine, la vindésine, la vinorelbine, les taxoides (paclitaxel et docétaxel)
- les anthracyclines comme notamment la doxorubicine, la daunorubicine, l'idarubicine, l'épirubicine, la mitoxantrone, la losoxantrone
- les topoisomérases des groupes I et II telles que l'étoposide, le teniposide, l'amsacrine, l'irinotecan, le topotecan et le tomudex
- les fluoropyrimidines telles que le 5-fluorouracile, l'UFT, la floxuridine
- les analogues de cytidine telles que la 5-azacytidine, la cytarabine, la gemcitabine, la 6-mercaptomurine, la 6-thioguanine
- les analogues d'adénosine telles que la pentostatine, la cytarabine ou le phosphate de fludarabine
- le méthotrexate et l'acide folinique
- les enzymes et composés divers tels que la L-asparaginase, l'hydroxyurée, l'acide trans-rétinoique, la suramine, la dexrazoxane, l'amifostine, l'herceptin ainsi que les hormones oestrogéniques, androgéniques
- les agents antivasculaires tels que les dérivés de la combretastatine ou de la colchicine et leurs prodrogues.

Il est également possible d'associer aux composés de la présente invention un traitement par des radiations. Ces traitements peuvent être administrés simultanément, séparément, séquentiellement. Le traitement sera adapté par le praticien en fonction du malade à traiter.

### Définitions

Le terme « halogène » fait référence à un élément choisi parmi F, Cl, Br, et I.

Le terme « alkyle » fait référence à un substituant hydrocarboné saturé, linéaire ou ramifié, ayant de 1 à 12 atomes de carbone. Les substituant méthyle, éthyle, propyle, 1-méthyléthyl, butyle, 1-méthylpropyl, 2-méthylpropyle, 1,1-diméthyléthyle, pentyle, 1-méthylbutyle, 2-méthylbutyle, 3-méthylbutyle, 1,1-diméthylpropyle, 1,2-diméthylpropyle, 2,2-diméthylpropyle, 1-éthylpropyle, hexyle, 1-méthylpentyle, 2-méthylpentyle, 1-éthylbutyle, 2-éthylbutyle, 3,3-diméthylbutyle, heptyle, 1-éthylpentyle, octyle, nonyle, décyle, undécyle, et dodécyle sont des exemples de substituant alkyle.

Le terme « alkylène » fait référence à un substituant hydrocarboné linéaire ou ramifié ayant une ou plusieurs insaturations, ayant de 2 à 12 atomes de carbone. Les substituants éthylènyle, 1-méthyléthylènyle, prop-1-ènyle, prop-2-ènyle, Z-1-méthylprop-1-ènyle, E-1-méthylprop-1-ènyle, Z-1,2-diméthyl-prop-1-ènyle, E-1,2-diméthylprop-1-ènyle, but-1,3-diényle, 1-méthylidènyl-prop-2-ènyle, Z-2-méthylbut-1,3-diényle, E-2-méthylbut-1,3-diényle, 2-méthyl-1-méthylidènylprop-2-ènyle, undéc-1-ènyle et undéc-10-ènyle sont des exemples de substituant alkylène.

Le terme « alkynyle » fait référence à un substituant hydrocarboné linéaire ou ramifié ayant au moins deux insaturations portées par une paire d'atomes de carbone vicinaux, ayant de 2 à 12 atomes de carbone. Les substituants éthynyle; prop-1-ynyle; prop-2-ynyle; et but-1-ynyle sont des exemples de substituant alkynyle.

Le terme « aryle » fait référence à un substituant aromatique mono- ou polycyclique ayant de 6 à 14 atomes de carbone. Les substituants phényle, napht-1-yle ; napht-2-yle ; anthracen-9-yl ; 1,2,3,4-tétrahydronapht-5-yle ; et 1,2,3,4-tétrahydronapht-6-yle sont des exemples de substituant aryle.

Le terme « hétéroaryle » fait référence à un substituant hétéroaromatique mono- ou polycyclique ayant de 1 à 13 atomes de carbone et de 1 à 4 hétéroatomes. Les substituants pyrrol-1-yle ; pyrrol2-yle ; pyrrol3-yle ; furyle ; thienyle ; imidazolyle ; oxazolyle ; thiazolyle ; isoxazolyle ; isothiazolyle ; 1,2,4-triazolyle ; oxadiazolyle ; thiadiazolyle ; tétrazolyle ; pyridyle ; pyrimidyle ; pyrazinyle ; 1,3,5-triazinyle ; indolyle ; benzo[b]furyle ; benzo[b]thiényle ; indazolyle ; benzimidazolyle ; azaindolyle ; quinoléyle ; isoquinoléyle ; carbazolyle ; et acridyle sont des exemples de substituant hétéroaryle.

Le terme « hétéroatome » fait référence ici à un atome au moins divalent, différent du carbone. N; O; S; et Se sont des exemples d'hétéroatome.

Le terme « cycloalkyle » fait référence à un substituant hydrocarboné cyclique saturé ou partiellement insaturé ayant de 3 à 12 atomes de carbone. Les substituants cyclopropyle; cyclobutyle; cyclopentyle; cyclopentènyle; cyclopentadiényle; cyclohexyle; cyclohexènyle; cycloheptyle; bicyclo[2.2.1]heptyle ; cyclooctyle; bicyclo[2.2.2]octyle; adamantyle ; et perhydronapthyle sont des exemples de substituant cycloalkyle.

Le terme « hétérocyclyle » fait référence à un substituant hydrocarboné cyclique saturé ou partiellement insaturé ayant de 1 à 13 atomes de carbone et de 1 à 4 hétéroatomes. De préférence, le substituant hydrocarboné cyclique saturé ou partiellement insaturé sera monocyclique et comportera 4 ou 5 atomes de carbone et 1 à 3 hétéroatomes.

Les avantages de l'invention seront plus particulièrement illustrés par les exemples suivants :

### Produit de départ : Bengamide naturel (BN) (Biosynthèse)

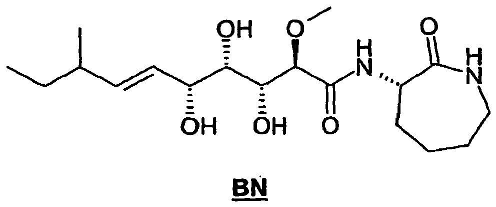

Le composé **BN** est obtenu selon la méthode décrite dans WO 2005/044803, et dont les étapes sont reprises ci-dessous :

### Etape 1 : Préparation d'une culture préliminaire de Myxococcus virescens ST200611 (DSM 15898) dans un erlenmeyer.

La souche de *Mixococcus virescens* ST200611 (déposée sous le numéro DSM 15898, selon les dispositions du Traité de Budapest, le 11/09/2003, au Deutsche Sammlung von Mikroorganismen und Zellkulturen GmbH (DSMZ), Mascheroder Weg 1b, 38124 Braunschweig, Germany). Les cellules végétatives de la souche DSM15898 ont une forme de bâtonnet, caractéristique de *Myxococcus virescens.* Sur des substrats nutritifs solides, *Myxococcus virescens* ST200611 (DSM 15898) forme des organes de fructification de couleur jaune-orange qui contiennent des myxospores rondes. Au lieu de la souche *Myxococcus virescens* ST200611 (DSM 15898), il est possible d'utiliser des mutants et/ou des variants qui synthétisent un ou plusieurs des produits selon l'invention.

La souche de *Mixococcus virescens* ST200611 est inoculée dans 100 mL d'une solution nutritive (1% de levure de boulanger fraîche, 1% de CaCl₂.2H₂O, HEPES 20mM, cyanocobalamine 0,00005%, pH 7,2) dans un erlenmeyer stérile de 300 mL, et le milieu de culture est incubé pendant 4 jours à 30°C sur un agitateur rotatif à 180 tours par minute. 5 mL de cette culture sont ensuite utilisés pour la préparation des cultures principales.

### Etape 2: Préparation d'une culture principale de Mixococcus virescens ST200611 (DSM 15898).

Un erlenmeyer stérile de 300 mL contenant 100 mL de la solution nutritive suivante (0,5% d'extrait de levure ; 0,5% de casitone ; 0,1% de CaCl₂.2H₂O ; 0,2% de MgSO₄.7H₂O, cyanocobalamine 0,00005%, pH 7,4) est inoculée avec 5 mL de la culture préliminaire préparée à l'étape 1, ou avec une culture qui s'est développée sur une plaque d'Agar fraîche (1% de levure de boulanger fraîche; 1% de CaCl₂.2H₂O ; HEPES 20mM, cyanocobalamine 0,00005%, pH 7,2 ; et 1,5% d'agar) et est incubée à 30°C sur un agitateur à 180 tours par minute. La production maximale de bengamide **BN** est atteinte après 72-96 heures.

### Etape 3 : Isolation du composé BN, à partir de cultures de Mixococcus virescens ST200611 (DSM 15898) obtenues à étape 2.

Le milieu de culture obtenu à l'étape 2 et reproduit à une échelle de 30 L de milieu de culture, est lyophilisé avec sa biomasse, et le lyophilisat est extrait avec 2 fois 5 L de méthanol. L'extrait méthanolique est évaporé partiellement sous pression réduite à un volume de 1,2 L, puis est chargé sur une colonne de 1,5 L de phase stationnaire CHP-20P (MCI® gel, 75-150 µ, Mitsubishi Chemical Corporation). La colonne est éluée avec du méthanol à 95%. Les fractions sont collectées et leur volume total est réduit à un volume de 1,5 L.

Cette dernière fraction est chargée sur une colonne Phenomenex Luna ® 10µ C18 (2) (taille : 50 mm x 250 mm) comportant une précolonne Luna ® 10µ C18 (2) (taille : 21,2 mm x 60 mm), puis est éluée (0,1% d'acétate d'ammonium, pH 4,6, ajusté avec de l'acide acétique) pendant 60 minutes avec un gradient de 5% à 95% d'acétonitrile dans l'eau. Le débit est de 150 mL/minute et le volume des fractions est de 200 mL. Des bengamides sont présents dans les fractions 6 à 14.

### Etape 4 : Purification du composé BN.

Les fractions obtenues à l'étape 3 sont lyophilisées et purifiées à nouveau par HPLC sur une colonne Phenomenex Luna ® 10µ C18 (2) (taille : 21 mm x 250 mm) possédant une précolonne Xterra® Prep MS C18 10µm (Waters, dimension : 19 x 10 mm). La colonne est éluée avec un gradient de 5% à 40% d'acétonitrile dans l'eau sur 40 minutes (en présence de 0,1 % d'acétate d'ammonium, pH 8,8, ajusté avec de la triethylamine), Les eliquotes (50 mL/minute) sont collectés par fractions de 7,5 mL. Les fractions 10-11 sont rassemblées et les solvants sont évaporés sous pression réduite pour fournir 145 mg de composé **BN** sous la forme d'un mélange 70/30 de diastéréoisomères, pureté >95%. Les diastéréoisomères sont des épimères en C16.

### Exemple 1: N-[(S)-1-H-2-oxo-perhydro-azépin-3-yl-]-[(E)-(2R,3R,4S,5R)-3,4,5-triacétoxy-2-méthoxy-8-méthyl-dec-6-ènamide

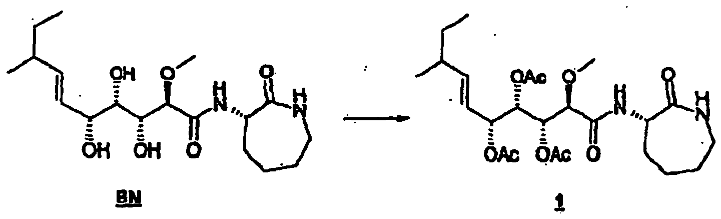

Dans un ballon de 10ml muni d'une agitation magnétique et sous atmosphère d'argon, on introduit successivement 1mL de CH2Cl2 (sur siliporite) et 47,7 mg (0,128 mmol) du composé **BN**. A à la solution ainsi obtenue, on ajoute 57 µL de pyridine, 8,1 mg de DMAP, et 60,4 µL d'anhydride acétique. La solution est agitée à température ambiante et sous argon. Après 2h30, le milieu réactionnel est hydrolysé par une solution aqueuse de NaHSO₄ 10%, et est extrait par un mélange AcOEt/nHeptane 4/1 (v/v)), La phase organique est séchée sur MgSO₄, filtrée, puis concentrée sous pression réduite. Le résidu obtenu est purifié par chromatographie sur silice SiO₂ 60 (25-40µm, Merck), éluant : CH₂Cl₂/MeOH (98/4 (v/v)). On recueille 56 mg (88%) du produit attendu 1 sous la forme d'un solide blanc.

Spectre RMN 1H (400MHz ) - δ en ppm - CDCl₃, mélange d'isomères : 0.86 (m, 3H) ; 0,98 (d, J = 6,5 Hz, 3H) ; de 1,25 à 1,60 (m, 4H) ; 1,90 (m, 2H) ; de 2,00 à 2.18 (m, 12H); de 3,25 à 3,44 (m, 2H): 3,45 (s, 3H); 3,84 (d, J = 5,0 Hz, 0,2H) et 3,86 (d, J = 5,0 Hz, 0,8H); 4,53 (m,1H) ; de 5,28 à 5,72 (m, 5H) ; 5,97 (m,1H) ; 7,88 (d, J = 6,0 Hz, 1H).

ES+/-: 499(+) = (M+H)(+); 521(+) = (M+Na)(+); 439(+) = (M+H)-OCOCH3 ; 997(+) = (2M+H)(+)

### Exemple 2; N-[(S)-1-benzyl-2-oxo-perhydro-azépin-3-yl]-(E)-(2R,3R,4S,5R)-3,4,5-triacétoxy-2-méthoxy-8-méthyl-dec-6-ènamide

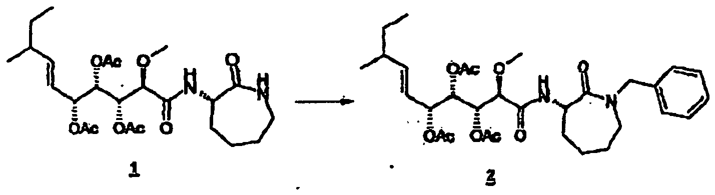

Dans un ballon de 10ml muni d'une agitation magnétique et sous atmosphère d'argon, on introduit successivement 1mL de DMF et 28,6 mg (63,15 µmol) du composé **1**. A à la solution ainsi obtenue, refroidie à 0°C, on ajoute 3,1 mg (1,2 éq.) de NaH à 50%. Le milieu réactionnel est agité pendant 5 minutes, puis on ajoute 12,64 µL (2 éq.) de bromure de benzyle. La réaction est poursuivie pendant 4 heures, puis le milieu réactionnel est hydrolysé par une solution aqueuse de NH₄Cl, puis extrait 2 fois par un mélange AcOEt/nHeptane (1/2). La phase organique est séchée sur MgSO₄, filtrée, puis concentrée sous pression réduite. Le résidu obtenu est purifié par chromatographie préparative (SiO₂ 60, Merck, éluant, CH₂Cl₂/MeOH (96/4)). On collecte 6,8 mg (21,5%) du produit **2** attendu.

Spectre RMN 1H (400MHz) - δ en ppm - CDCl₃ mélange d'isomères: 0,82 (m, 3H) ; 0,94 (d, J = 6,5 Hz, 3H) ; de 1,10 à 2,15 (m, 18H) ; 3,25 (dd, J = 5,5 et 14,5 Hz, 1H) ; 3,42 (s, 0,6H) et 3,43 (s, 2,4H) ; 3.51 (dd, J = 11.5 et 14.5 Hz, 1H) : 3.81 (d, J = 5.0 Hz, 0,2H) et 3,83 (d, J = 5.0 Hz, 0,8H) ; 4,55 (d. J = 14,5 Hz, 1H) ; 4,63 (m, 1H) ; 4,74 (d, J = 14,5 Hz, 1H) ; de 5,25 à 5,68 (m, 5H) ; de 7.23 à 7.37 (m, 5H) ; 8,05 (d, J = 6,0 Hz, 1H).

ES+/- : 589(+) = (M+H)(+) ; 529(+) = (M+H)(+)-OAc ; 1117(+) = (2M+H)(+)

### Exemple 3: N-[(S)-1-benzyl-2-oxo-perhydroazépin-3-yl]-(E)-(2R,3R,4S,5R)-3,4,5-Trihydroxy-2-méthoxy-8-méthyl-dec-6-ènamide

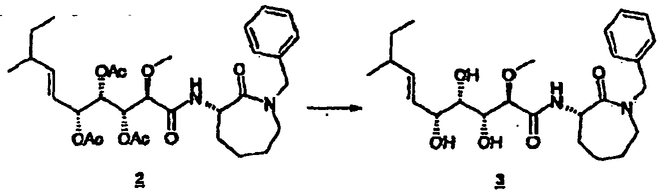

Dans un ballon de 5 ml muni d'une agitation magnétique, on introduit successivement 500 µL de méthanol, 6,8 mg (11,55 µmol) du composé **2**, et 4,8 mg (3 éq.) de carbonate de potassium. Le milieu réactionnel est concentré sous pression réduite après 1h10 de réaction, puis le résidu est repris dans du CH₂Cl₂. L'insoluble est filtré et lavé 3 fois avec CH₂Cl₂. Le filtrat est collecté, les solvants sont évaporés, sous pression réduite et le résidu est séché sous vide. 5,8 mg de produit brut sont alors recueillis. Ce produits brut est purifié par CCM (SiO₂ 60, Merck. éluant, CH₂Cl₂/MeOH (96/4)). On collecte 3,8 mg (71%) du produit 3 attendu.

Spectre RMN 1H (400MHz) - δ en ppm - CDCl₃, mélange d'isomères : 0,86 (m, 3H) : 1,00 (d, J = 6,5 Hz, 0,8H) et 1.02 (d, J = 6,5 Hz, 2,2H) ; de 1,15 à 2,15 (m, 9H) ; de 3.00 à 3,30 (m large, 3H) ; 3,48 (dd, J = 11,5 et 14,5 Hz, 1H) ; 3,58 (s, 3H) ; 3.65 (m large, 1H) ; 3.81 (d, J = 6,5 Hz, 1H) ; 3,88 (d large, J = 6,5 Hz, 1H) ; de 4,22 à 4.80 (m, 5H) ; 5,49 (dd, J = 6,5 et 15,5 Hz, 1H) ; 5,72 (m, 1H) ; de 7,22 à 7,39 (m, 5H) : 8,20 (d large, J = 8,0 Hz, 1H).

ES+/- : 463(+)=(M+H)(+) ; 461(-)=(M-H)(-)

### Exemple 12: N-[(S)-1-(4-fluoro-benzyl)-2-oxo-perhydro-azépin-yl)-(E)-(2R,3R,4S,5R)-3,4,5-triacétoxy-2-méthoxy-8-méthyl-dec-6-ènamide

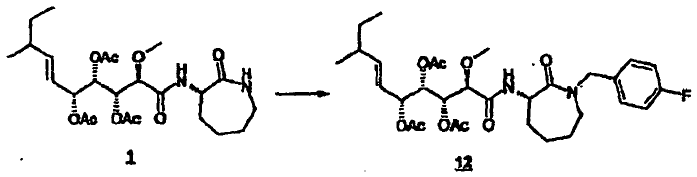

Dans un ballon de 10ml muni d'une agitation magnétique et sous atmosphère d'argon, on introduit successivement 1 mL de 4-methyl-2-pentanone et 50 mg (100 µmol) du composé **1**, 95mg (500µmol) de bromure de 4-fluoro benzyle et 163 mg (500µmol) de carbonate da césium anhydre. Le milieu réactionnel est chauffé pendant 24h à 50°C. On laisse revenir à la température ambiante, puis on coule dans la suspension 10ml d'acétate d'éthyle et 5ml d'une solution aqueuse saturée de chlorure d'ammonium.. Après décantation la phase aqueuse est réextraite 2 fois avec 10 ml d'acétate d'éthyle. Les phases organiques sont réunies, séchées sur sulfate de sodium. Après filtration et évaporation à sec, on obtient 300 mg de produit brut, qui est purifié par chromatographie préparative (SiO₂ 60, Cartouche Blotage 20g 40-60µm, éluant, AcOEt/Heptane (55/45). On obtient 45mg (74%) du produit **12** attendu (Rf : 0,30).

Spectre RMN 1H (400MHz) - δ en ppm - DMSO-d₆, mélange d'isoméres ( 70 % - 30 %) : 0,76 (t, J = 7,5 Hz, 0,9H) ; 0,79 (t, J = 7,5 Hz, 2,1H) ; 0,88 (d, J = 7.0 Hz, 3H) : 1,08 (m, 1H); de 1,17 à 1,30 (m, 2H) ; 1,37 (m, 1H) ; 1.65 (m, 2H) ; 1,81 (m, 2H) ; de 1,94 à 2,00 (m masqué, 1H) ; 1,97 (s, 3H) ; de 2,01 à 2,04 (m, 8H) ; de 3,21 à 3,30 (m partiellement masqué, 1H) ; 3,33 (s, 3H) ; 3,57 (dd, J =10,5 et 15,0 Hz, 1H); 3,83 (d, J = 4,5 Hz, 0,3H) ; 3,84 (d, J = 4,5 Hz, 0,7H) ; 4,50 (d, J = 14,5 Hz, 1H) ; 4,55 (m, 1H) ; 4,60 (d, J = 14,5 Hz, 1H) ; de 5,20 à 5,39 (m, 4H) ; 5,55 (m, 1H) ; 7,13 (t large, J = 9,0 Hz, 2H) ; 7,33 (dd large, J = 6,0 et 9,0 Hz, 1H) ; 7,99 (d, J = 6,5 Hz, 1H).

ES: 607(+)=(M+H)(+); 547(+)=(M+H)(+)-CH₃COOH

### Exemple 13: N-[(S)-1-(4-fluoro-benzyl)-2-oxo-perhydro-azépin-3-yl]-(E)-(2R,3R,4S,5R)-3,4,5-Trihydroxy-2-méthoxy-8-méyhyl-dec-6-énamide

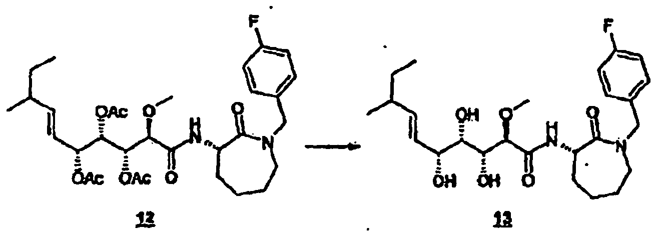

Dans un ballon de 10 ml muni d'une agitation magnétique, on introduit successivement 3,0 ml de méthanol, 33 mg (54 µmol) du composé **12** et 24 mg (173 µmol, 3,2 éq.) de carbonate de potassium. Le milieu réactionnel est agité 2h à température ambiante, puis concentré, extrait par 3 fois 5ml de chlorure de méthylène, lavé avec 3ml d'eau, puis avec une solution saturée de chlorure de sodium. Les phases organiques réunies sont séchées sur surate de sodium, filtrées et evaporées à sec, on obtient 23 mg (89%) de produit **13** attendu.

Spectre RMN 1H (400MHz) - δ en ppm - DMSO-d6, mélange d'isomères ( 70 % - 30 %) : 0,82 (t, J = 7,5 Hz, 2,1H); 0,84 (t, J = 7,5 Hz, 0,9H) ; 0,93 (d, J = 7,0 Hz, 0,9H) ; 0,94 (d, J = 7,0 Hz, 2,1H); 1,08 (m,1H) ; de 1,20 à 1,31 (m, 2H) ; 1,38 (m, 1H): 1,65 (m, 2H) 1,79 (m, 1H) ; 1,88 (m, 1H) ; 1,89 (m, 1H) ; de 3,16 à 3,33 (m masqué, 4H) ; 3,38 (d large, J = 7,0 Hz, 1H) ; 3,57 (dd, J = 11,0 et 15,0 Hz, 1H) : 3,81 (dd, J = 2,5 et 7,5 Hz, 1H) ; 3,71 (d, J = 7,0 Hz, 1H) ; 3,99 (t, J = 7,0 Hz, 1H) ; de 4,08 à 4,77 (m très étalé, 3H) ; 4,50 (d, J = 14,5 Hz, 1H) ; 4,57 (d, J = 14,5 Hz, 1H) ; 4,61 (m, 1H) ; 6,38 (m, 1H) ; 5,49 (dd, J = 7,0 et 15,0 Hz, 1H) ; 7,15 (t large, J = 9,0 Hz, 2H) ; 7,32 (dd large, J = 5,5 et 9,0 Hz, 2H) ; 7,89 (d, J =6,3 Hz, 1H).

IC: 481(+)=(M+H)(+)

### Exemple 14: N-{(S,-1-(3,5-difluoro-benzyl)-2-oxo-perhydro-azépin-3-yl]-(E)-(2R,3R,4S,5R)-3,4,5-triacétoxy-2-méthoxy-8-méthyl-dec-6-ènamide

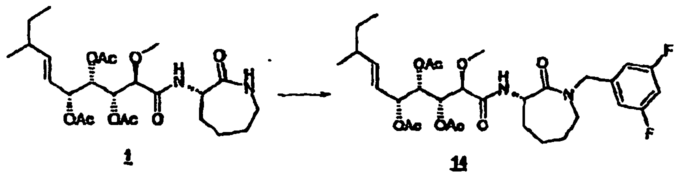

Dans un ballon de 10ml muni d'une agitation magnétique et sous atmosphère d'argon, on introduit successivement 1 mL de 4-méthyl-2-pentanone et 50 mg (100 µmol) du composé **1**, 104mg (602µmol) de bromure de 3,5-difluoro benzyle et 163 mg (502µmol) de carbonate de césium anhydre. Le milieu "réactionnel est chauffé pendant 24h à 50°C. On laisse revenir à la température ambiante, puis on coule dans la suspension 10ml d'acétate d'éthyle et 5ml d'une solution aqueuse saturée de chlorure d'ammonium. Après décantation, la phase aqueuse est réextraite 2 fois avec 10 ml d'acétate d'éthyle. Les phases organiques sont réunies, séchées sur sulfate de magnésium. Aprés filtration et évaporation à sec, on obtient 290 mg de produit brut, qui est purifié par chromatographie préparative (SiO₂ 60, Cartouche Biotage 20g 40-60µm, éluant, AcOE/Heptane (55/45). On obtient 35mg (56%) du produit **14** attendu (Rf : 0,25).

Spectre RMN 1H (400MHz) - δ en ppm - DMSO-d6, mélange d'isomères ( 70 % - 30 %) : 0,75 (t J = 7,5 Hz, 0,9H) : 0,76 (t, J = 7,5 Hz, 2,1H) ; 0,87 (d, J = 7.0 Hz, 3H) ; de 1,11 à 1,30 (m, 3H) ; 1,39 (m, 1H) ; 1,69 (m, 2H) ; 1.84 (m, 2H) ; de 1,92 à 2,00 (m masqué, 1H) ; 1,97 (s, 3H); de 2,00 à 2,04 (m, 6H) ; 3,25 (m partiellement masqué, 1H) ; 3,33 (s, 3H); 3,63 (dd, J = 11,5 et 15,5 Hz, 1H) ; 3,84 (d, J = 4,5 Hz, 0,3H) ; 3,85 (d, J = 4,5 Hz, 0.7H) ; 4,40 (d, J = 15,5 Hz. 1H) ; 4,60 (m, 1H) ; 4,76 (d, J = 15,5 Hz, 1H) ; de 5.21 à 5,39 (m, 4H) ; 5,54 (m, 1H); de 6,96 à 7,03 (m, 2H) ; 7,11 (tt, J = 2,5 et 9,5 Hz, 1H) ; 7,98 (d, J = 6,5 Hz, 1H).

ES: 625(+)=(M+H)(+) ; 585(+)=(M+H)(+) -CH₃COOH

### Exemple 15: N-[(S)-2-oxo-1-(3,5-difluorobenzyl)-perhydro-azépin-3-yl]-(E)-(2R,3R,4S,5R)-3,4,5-Trihydoxy-2-méthyl-dec-6-ènamide

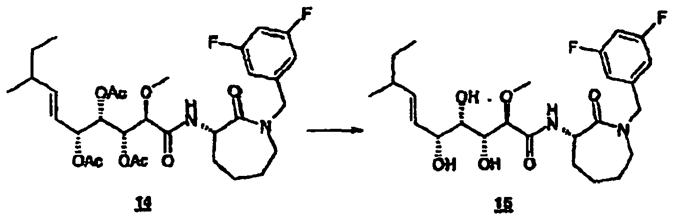

Dans un ballon de 10 ml muni d'une agitation magnétique, on introduit successivement 3 ml de méthanol, 32 mg (51 µmol du composé **14** et 22 mg (159µmol. 3,1 éq.) de carbonate de potassium. Le milieu réactionnel est agité 3h à température ambiante, puis repris dans d'acétate d'éthyl et une solution aqueuse saturée de chlorure d'ammonium. Après décantation, la phase organique est séchée sur sulfate due sodium. Après filtration et évaporation à sec, on obtient 25 mg (98%) du produit **15** attendu.

Spectre RMN 1H (400MHz ) - δ en ppm - DMSO-d6, mélange d'isomères ( 70 % - 30 %) : 0,81 (t, J= 7,5 Hz, 2,1H) ; 0,82 (t J = 7,5 Hz, 0,9H) ; 0,92 (d. J = 7,0 Hz. 0.9H) ; 0,93 (d. J = 7,0 m 2.1H) ; de 1,08 à 1,48 (m, 4H) ; 1,67 (m, 2H) ; de 1.77 à 1,92 (m, 2H) ; 1.99 (m, 1H); de 3.19 à 3,39 (m masqué, 2H) ; 3,27 (s, 3H) ; 3,59 (dd, J = 2,5 et 7.5 Hz, 1H) ; 3,84 (dd, J = 11.0 et 15.0 Hz, 1H) ; 3.71 (d, J = 7,5 Hz. 1H) ; 3.98 (t, J = 7,0 Hz, 1H); de 4.13 à 4.73 (m très étalé, 3H) ; 4,43 (d, J = 18,5 Hz, 1H) ; 4.66 (m, 1H) ; 4.70 (d, J =15,5 Hz. 1H) ; 5,38 (m, 1H); 5,48 (dd, J =7,5 et 15.5 Hz, 1H); 8,99 (m. 2H) : 7.13 (tt, J = 2,5 et 9.5 Hz, 1H); 7,92 (d, J= 6,5 Hz, 1H).

ES: 499(+)=(M+H)(+)

### Exemple 16: N-{(S)-1-(3,4-difluoro-bennzyl)-2-oxo-perhydro-azépin-3-yl]-(E)-(2R,3R,4S,5R)-3,4,5-triacétoxy-2-méthoxy-méthyl-dec-6-ènamide

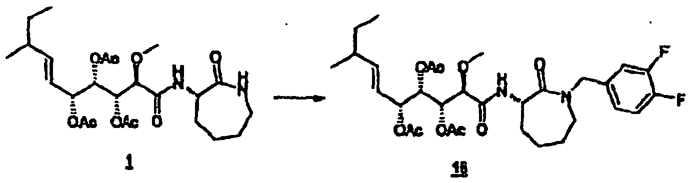

Dans un ballon de 10ml muni d'une agitation magnétique et sous atmosphère d'argon, on introduit successivement 1,5 mL de 3-methyl-2-butanone et 70 mg (140 µmol) du composé 1, 146mg (700µmol) de bromure de 3,4-difluoro benzyle et 230 mg (700µmol de carbonate de césium anhydre. Le milieu réactionnel est chauffé pendant 48h à 80°C. On laisse le milieu reactionnel revenir à la température ambiante, on coule dans la suspension 10ml de chlorure de méthylène et 5ml d'eau, après decantation, la phase organique est séchée sur sulfate de sodium. Après filtration et évaporation à sec, on obtient 165 mg de produit brut, qui est purifié par chromographie préparative (SiO₂ 60, Cartouche 8g 40-60µm, éluant, AcOEt/Heptane (50/50). On obtient 25 mg 29%) du produit 16 attendu (Rf : 0,15)

Spectre RMN 1H (400MHz ) - δ en ppm - DMSO-d6, mélange d'isomères ( 70 % - 30 %) 0,75 (t. J = 7,5 Hz, 0,9H) : 0,78 (t, J = 7,5 Hz, 2,1H) : 0,87 (d. J = 7,0 Hz, 3H) ; de 1,00 à 1,28 (m, 3H) : 1,38 (m, 1H) ; 1,67 (m 2H) ; 1,82 (m. 2H) ; de 1,93 à 2.00 (m masqué. 1H) ; 1,97 (s, 3H) ; de 2,01 à 2,04 (m, 6H) ; de 3,21 à 3,48 (m partiellement masqué, 1H) ; 3,32 (s, 3H) ; 3.58 (dd. J = 11,5 et 15,0 Hz, 1H) ; 3.83 (d, J = 4,5 Hz, 0,3H); 3,84 (d, J = 4,5 Hz, 0,7H) ; 4,43 (d, J = 15,0 Hz, 1H) ; 4,56 (m, 1H); 4,67 (d, J =15,0 Hz.1H) ; de 5,20 à 5,38 (m, 4H) ; 5,53 (m, 1H) ; 7,13 (m, 1H) ; de 7,29 à 7,41 (m, 2M) ; 7,98 (d, J = 6,5 Hz 1H).

IC: 642(+)=(MH₄)(+) ; 565 (+)=(M+H)(+) -CH₃COOH

### Exemple 17: N-[(S)-1-(3,4-difluobenzyl)-2-oxo-perhydro-azépin-3-yl-(E)-(2R,3R,48,5R)-3,4,5-Trihydroxy-2-méthoxy-8-méthyl-dec-6-ènamide

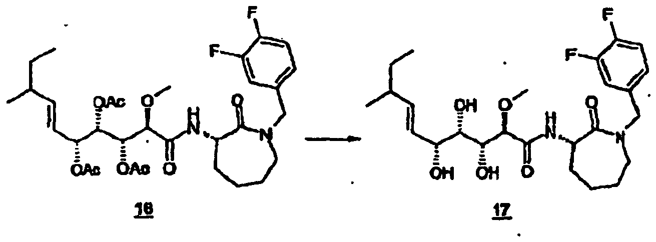

Dans un ballon de 10 ml muni d'une agitation magnétique, on introduit 1 successivement 3,0 ml de métharrol, 25 mg (40 µmol) du composé **16** et 18 mg (130 µmol, 3,3 éq.) de carbonate de potasse. Le milieu réactionnel est agité 3h à tempétature ambiante, puis extrait avec chlorure de méthylène, lavé à l'eau, après séchage sur sulfate de sodium, filtration et évaporation à sec, on obtient 17 mg (85%) de produit **17** attendu.

ES. 499(+)=M+H)(+)

### Exemple 18 : N-[(S)-2-oxo-1-(2,3,5,6-tétrafluoro-benzyl)-perhydro-azépin-3-yl)-(2R,3R,4S,5R)-3,4,5-triacétoxy-2-méthoxy-8-méthyl-dec-6-ènamide

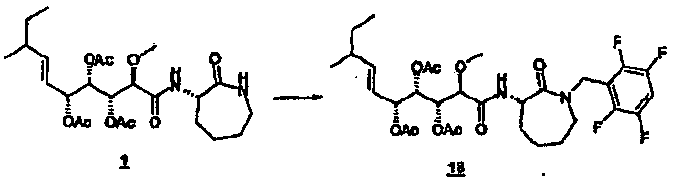

Dans un ballon de 10ml muni d'une agitation magnétique et sous atmosphère d'argon, on introduit successivement 2 mL de 3-méthyl-2-butanone et 100 mg (200 µmol) du composé **1**, 199 mg (1,0mmol) de bromure de 2,3,5,6-tetrafluoro benzyle et 327mg (1,0 mmol) de carbonate de césium anhydre. Le milieu réactionnel est chauffé pendant 24h à 80°C. On laisse le milieu réactionnel revenir à ta température ambiante, on coule dans la suspension 10ml de chlorure de méthylène et 5ml d'eau, après décantation, la phase organique est séchée sur sulfate de sodium. Après filtration et évaporation à sec, on obtient 125 mg de produit brut; qui est purifié par chromatogtaphie préparative (SiO₂ 60, Cartouche Blotage 8g 40-80µm, éluant, AcOEt/Heptane (50/50). On obtient 15mg(11%)du produit **18** attendu (Rf : 0.20).

Spectre RMN 1H (400MHz ) - δ en ppm - DMSO-d6, mélange d'isomères ( 70 % - 30 %) : 0,76 (t, J = 7,6 Hz. 3H) ; 0,87 (t. J = 7.0 Hz. 0,9H); 0,88 (d, J = 7,0 Hz, 2,1H); de 1,06 à 1,41 (m, 4H) : de 1,51 à 1,87 (m masqué. 4H); de 1,91 à 2.05 (m, 7H); 1,92 (s, 3H) ; de 3,15 à 3.55 (m partiellement masqué, 1H) : 3.29 (s, 3H); 3,64 (m. 1H) ; 3,81 (d. J = 4,5 Hz, M : 4,52 (m, 1H) : de 4,07 à 4,76 (m. 2H) ; de 5,19 à 5,37 (m, 4H): 5,51 (m, 1H) ; 7.84 (m, 1H); 7,94 (d, J = 6,5 Hz, 1H).

ES: 561 (+)=(M+H)(X+): 601 (+)=(M+H)(+) -CH₃COOH

### Exemple 19: N-[(S)-1-(2,3,5,8-tétrafluoro-benzyl)-2-oxo-perhydro-azépin-ènamide

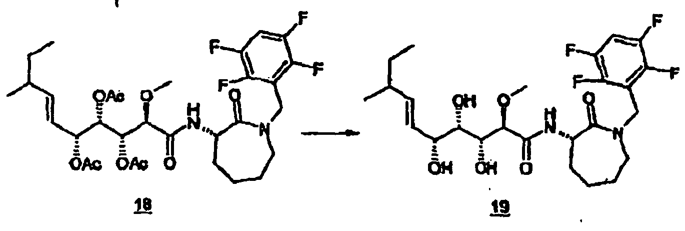

Dans un ballon de 10 ml muni d'une agitation magnétique, on introduit successivement 3,0 ml de méthanol. 22mg (33 µmol) du composé **18** et 14mg (100 µmol, 3,0 éq.) de carbonate de potassium. Le milieu réactionnel est agité 2h à température ambiante, puis extrait avec du chlorure de méthylène, et lavée à l'eau. Après séchage sur sulfate de sodium, filtration et évaporation à sec, on obtient 15 mg (84%) de produit **19** attendu.

Spectre RMN 1H (400MHz) - δ en ppm - DMSO-d6, mélange d'Isomères ( 70 % - 30 %) : 0.79 (t. J = 7,5 Hz. 2,1H) ; 0,81 (t, J = 7,5 Hz. 0,9H), 0,91 (d, J = 7.0 Hz, 0,9H); 0,93 (d, J = 7,0 Hz. 2,1H) ; de 1,10 à 1,42 (m, 4H); de 1,57 à 1,81 (m, 4H) ; 1,98 (m, 1H) ; de 3,16 à 3,40 (m partiellement masqué, 2H) ; 3,25 (s, 3H) ; de 3,53 à 3,72 (m. 3H) ; 3,96 (m, 1H); 4.36 (d. J = 6,0 Hz. 1H); 4,42 (m, 1H) : 4,56 (m, 2H) ; 4,66 (d. J = 15.0 Hz. 1H) : 4.74 (d, J = 15,0 Hz, 1H); 5,35 (m, 1H) : 5.47 (m, 1H) ; de 7,79 à 7.89 (m. 2H).

ES: 535(+)=(M+H)(+)

### Exemple 20 : N-[(S;2-oxo-1-(2,3,4,5,6-pentafluoro-benzyl)-perhydro-azépin-3-yl)-(E)-(2R,3R,4S,5R)-3,4,5-triacetoxy-2-méthoxy-8méthyl-dec-6-ènamide

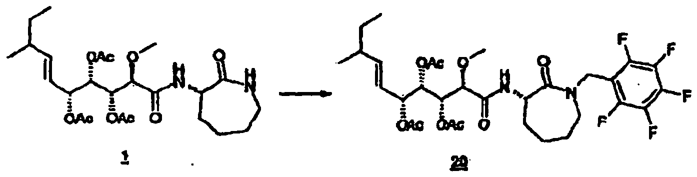

Dans un ballon de 10ml muni d'une agitation magnétique et sous atmosphère d'argon, on introduit successivement 1,5 mL de 3-méthyl-2-butanone et 60 mg (120 µmol) du composé 1, 172 mg (659mmol) de bromure de 2,4,5,6-pentafluoro benzyle et 196mg (802 mmol) de carborate de césium anhydre. Le milieu réactionnel est chauffé pendant 12h à 50°C. Puis, 172 mg (659mmol) de bromure de 2,3,4,5,6-petafluoro benzyle et 196mg (602 mmol) de carbonate de césium anhydre son rajouté dans le milieu qui est chauffé pendant 48h, et de nouveau 172 mg (659mmol) de bromure de 2,3,4,5,6-pentafluoro benzyle et 19emg (602 mmol) de carbonate de césium anhydre son rajoute dans le milieu qui est chauffé pendant 12h à 50°C, Dans la suspension est coûte do l'acétate d'ethyle et de l'eau, après décantation, la phase organique est séchée sur sulfate de Magnésium. Après filtration et évaporation à sec, le produit brut est purifié par chromatographie préparative (SiO₂ 60, Cartouche RS 4g 40-80µm, éluant, AcOEt/Heptane (55/45). On obtient 29mg (35%) du produit **20** attendu (Rf : 0.28).

Spectre RMN 1H (400MHz ) - δ en ppm - DMSO-d6, mélange d'isomères ( 70 % - 30 %) : 0,81 (t, J = 7,5 Hz, 0,9H); 0,82 (t, J = 7,5 Hz, 2,1H); 0,92 (d, J = 7,0 Hz, 0,9H) : 0.94 (d. J = 7,0 Ha 2,1H): de 1.12 à 1,64 (m partiellement masqué, 4H); de 1,75 à 1,95 (m, 3H); 2,01 (m partiellement masqué, 1H); 2,03 (s. 3H) ; 2,05 (s, 3H) ; 2,09 (s, 0,9H) ; 2.10 (s. 2,1H) ; 3,31 (m, 1H): 3,40 (s, 3H) ; 3,59 (m, 1H); 3.78 (d, J = 4,5 Hz, 0,3H); 3,80 (d, J = 4,5 Hz, 0,7H) ; 4,57 (m, 1H) ; 4,71 (d, J = 15,5 Hz, 1H) ; 4,78 (d, J =15,5 Hz, 1H) ; de 5,23 à 5,43 (m, 3H) ; 5,53 (m, 2H) ; 5,62 (dd, J = 7,5 et 15,5 Hz. 1H) ; 7,91 (d, J = 6,5 Hz, 1H)..

ES: 679(+)=(M+H)(+); 619(+)=(M+H)(+) -CH₃COOH

### Exemple 21: N-[(S)-1-(2,3,4,5,8-pentafluorobenzyl-Z-oxo-perhydro-azépin-3-yl]-(E)-[2R,3R,4S,5R]-3,4,5-Trihydroxy-2-méthoxy-8-méthyl-dec-6-ènamide

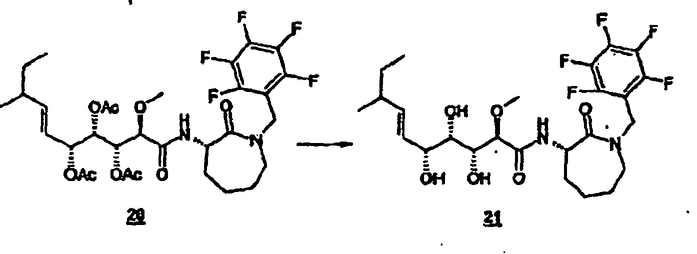

Dans un ballon de 10 ml muni d'une agitation magnétique, on introduit successivement 2,50 ml de méthanol, 28.5mg (42 µmol) du composé **20** et 18 mg (130µmol, 3,1 éq.) de carbonate de potassium. Le milieu réactionnel est agité 2h à température ambiante, puis extrait avec de l'acétate d'éthyle, lavé à l'eau. Après séchage sur sulfate de magnésium, filtration et évaporation à sec, on obtient 13 mg (57%) de produit **21** attendu.

Spectre RMN 1H (400MHz) - δ en ppm - DMSO-d6, mélange d'isomères ( 70 % - 30 %) : 0,79 (t, J = 7,5 Hz, 2,1H) ; 0,81 (t, J = 7,5 Hz, 0,9H) ; 0,91 (d, J = 7,0 Hz, 0.9H) ; 0,93 (d, J = 7,0 Hz, 2,1H) ; de 1,11 à 1,40 (m, 4H) ; de 1.57 à 1,90 (m, 4H) ; 1,98 (m, 1H) ; de 3,23 à 3,36 (m partiellement masqué, 2H) ; 3,25 (s, 3H) ; de 3,52 à 3,68 (m, 2H) ; 3,70 (d, J = 7,5 Hz, 1H); 3,96 (m, 1H) ; 4,35 (d, J = 5,5 Hz, 1H); 4,40 (m, 1H) ; de 4,52 à 4,59 (m, 2H) ; de 4,64 à 4,72 (m, 2H) ; 5,36 (m, 1H) ; 5,46 (dd, J = 7,5 et 15,5 Hz, 1H) ; 7,84 (d, J = 8,5 Hz, 1H).

ES: 553(+)=(M+H)(+)

### Exemple 22: N-[(S)-2-oxo-1-(4-cyano-3-fluoro-benzyl)-perhydro-azépin-3-yl]-(E)-(2R,3R,4S,5R)-3,4,5-triacétoxy-2-méthoxy-8-méthyl-dec-6-ènamide

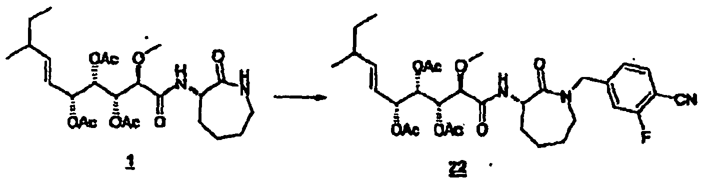

Dans un ballon de 50 ml muni d'une agitation magnétique et sous atmosphère d'argon, on introduit successivement 15 mL de 3-méthyl-2-butanone et 800 mg (1,6 mmol) du composé **1**, 1,72 g (8,0 mmol) de bromure de 4-cyano-3-fluoro-benzyle et 2,61 g (8,0 mmol) de carbonate de césium anhydre. Le milieu réactionnel est chauffé pendant 24h à 50°C. Dans la suspension est coulé de l'acétate d'éthyle (100 ml) et de l'eau (70 ml), après décantation, la phase organique est lavé avec 70 ml d'eau, séchée sur sulfate de Magnésium. Après filtration et évaporation à sec, le produit brut (2,4 g) est purifié par chromatographie préparative (SiO₂ 60, Cartouche RS 20g 40-60µm, éluant, AcOEt/Heptane en gradient. On obtient 227 mg (22%) du produit **22** attendu.

ES: 654(+)=(M+Na)(+)

Spectre RMN 1H (400MHz ) - δ en ppm - DMSO-d6, mélange d'isomères (80 % - 20 %) : 0,74 (t, J = 7,5 Hz, 0,6H) ; 0,75 (t, J = 7,5 Hz, 2,4H) ; 0,88 (d, J = 7,0 Hz, 2,4H) ; 0,87 (d, J = 7,0 Hz, 0,6H) ; de 1,12 à 1,29 (m, 3H); 1,42 (m, 1H) ; de 1,57 à 1,76 (m, 2H) ; de 1,79 à 1,88 (m, 2H) ; de 1,90 à 2,00 (m, 1H) ; 1,98 (s, 2,4H) ; 1,97 (s, 0,6H) ; 2,01 (s, 2,4H) ; 2,02 (s, 0,6H) ; 2,03 (s, 0.6H) ; 2,04 (s, 2,4H) ; de 3,22 à 3,35 (m partiellement masqué, 1H) ; 3,31 (s, 0,6H) ; 3,32 (s, 2,4H) ; 3,66 (m, 1H) ; 3,84 (d, J = 4,5 Hz, 0,2H) ; 3,85 (d, J = 4,5 Hz, 0,8H) ; 4,49 (d, J = 16,0 Hz, 1H) ; 4,61 (m, 1H) ; 4,85 (d, J = 16,0 Hz, 1H) ; de 5,20 à 5,38 (m, 4H) ; 5,52 (dd, J = 8,0 et 15,5 Hz, 0,8H) ; 5,54 (dd, J = 8,0 et 15,0 Hz, 0,2H) ; 7,32 (dd, J = 1,5 et 8,0 Hz, 1H) ; 7,41 (dd, J = 1,5 et 10,5 Hz, 1H) ; 7,88 (dd, J = 7,0 et 8,0 Hz, 1H) : 7,97 (d, J = 6,5 Hz, 1H).

### Exemple 23 : N-[(S)-1-(4-cyano-3-fluorobenzyl-2-oxo-perhydro-azépin-3-yl]-(E)-(2R,3R,4S,5R)-3,4,5-Trihydroxy-2-méthoxy-8-méthyl-dec-6-ènamide

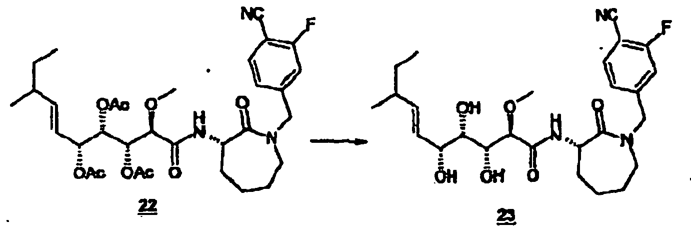

Dans un ballon de 50 ml mml d'une agitation magnétique, on introduit successivement 8,0 ml de méthanol, 128 mg (203 µmol) du composé **22** et 90 mg (648 µmol, 3,1 éq.) de carbonate de potassium. Le milieu réactionnel est agité 10 minutes à température ambiante, versé dans 20 ml d'une solution saturée de NH₄Cl, puis extrait avec de l'acétate d'éthyle (2 fois 25 ml). La phase aqueuse est saturée avec NaCl, et extraite avec 20 ml de CH₂Cl₂. Les phases organiques sont réunies et évaporées à sec, on obtient 164 mg de produit brut, qui est chromatographié sur une cartouche de silice (9g, éluant, CH₂Cl₂/isopropanol en gradient : Isopropanol 0 à 10%) pour donner 77 mg (75%) de produit 23 attendu.

ES: 506(+)=(M+H)(+)

Spectre RMN 1H (400MHz ) - δ en ppm - DMSO-d6, mélange d'isomères (80 % - 20 %): 0,80 (t, J = 7,5 Hz. 2,4H) ; 0,82 (t, J = 7,5 Hz, 0,6H) ; 0,92 (d, J = 7,0 Hz, 0,6H) ; 0,93 (d, J = 7,0 Hz, 2,4H) ; de 1,11 à 1,32 (m, 3H) ; 1,42 (m, 1H) ; de 1,60 à 1,74 (m, 2H) ; de 1,77 à 1,92 (m, 2H) ; 1,98 (m, 1H) ; de 3,21 à 3,38 (m partiellement masqué, 2H) ; 3,21 (s, 3H) ; de 3,55 à 3,74 (m, 2H) ; 3,71 (d, J = 7,5 Hz, 1H) ; 3,97 (m, 1H) ; 4,37 (d, J = 6,0 Hz, 1H) ; 4,42 (m, 1H) ; 4,50 (d, J = 16,0 Hz, 1H) ; 4,55 (d, J = 4,5 Hz, 1H); 4,88 (m, 1H) ; 4.79 (d, J = 16,0 Hz, 1H) ; 5,37 (m, 1H) ; 5,48 (dd, J = 7,5 et 15,5 Hz, 1H); 7,30 (dd, J = 1,5 et 8,0 Hz, 1H) ; 7,70 (dd, J = 1,5 et 10,5 Hz, 1H); de 7,87 à 7,93 (m, 2H).

### Exemple 24: N-[(S)-2-oxo-1-(3-cyano-4-fluoro-benzyl)-perhydro-azépin-3-yl-(E)(2R,3R,4S,5R)-3,4,5-triacétoxy-2-méthoxy-8-méthyl-dec-6-ènamide

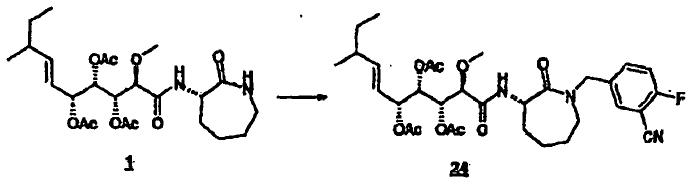

Dans un ballon de 50 ml muni d'une agitation magnétique et sous atmosphère d'argon, on introduit successivement 10 mL de 3-méthyl-2-butanone et 630 mg (1,26 mmol) du composé **1**, 1,35 g (6,3 mmol) de bromure de 3-cyano-4-fluoro-benzyle et 2,05 g (6,3 mmol) de carbonate de césium anhydre. Le milieu réactionnel est chauffé pendant 24h à 50°C. Le milieu réactionnel est laissé revenir à TA. filtré sur fritté, et évaporé à sec, le produit bruit (1,83 g) est purifié par chromatographie preparative (SiO₂ 60, Cartouche RS 20g 40-60µm, éluant AcOEt/Heptane en gradient. On obtient 298 mg (47%) du produit **24** attendu.

ES: 654(+)=(M+Na)(+)

Spectre RMN 1H (400MHz) - δ en ppm - DMSO-d6, mélange d'isomères (80 % - 20 %) : 0,74 (t, J = 7,5 Hz, 0,6H) ; 0,75 (t, J = 7,5 Hz, 2,4H) ; 0,86 (d, J = 7,0 Hz, 2,4H) ; 0,87 (d, J = 7,0 Hz, 0,6H) ; de 1,10 à 1,28 (m, 3H); 1,41 (m, 1H); de 1,58 à 1,74 (m, 2H); de 1,78 à 1,88 (m, 2H); de 1,90 à 2,00 (m partiellement masqué, 1H) ; 1,97 (s, 2,4H) ; 1,98 (s, 0,6H) ; 2,01 (s, 2,4H) ; 2,02 (s, 0,8H) ; 2,03 (s, 0,6H) ; 2,04 (s, 2,4H) ; de 3,23 à 3,36 (m partiellement masqué, 1H); 3,32 (s, 0,6H) ; 3,33 (s, 2,4H) ; 3,62 (m, 1H); 3,85 (d, J = 4,5 Hz, 0,2H) ; 3,86 (d, J = 4,5 Hz, 0,8H) ; 4,42 (d, J = 15,0 Hz, 1H) ; 4,59 (m, 1H) : 4,77 (d, J = 16,0 Hz, 1H) ; de 5,21 à 5,38 (m, 4H); 5,63 (dd, J = 8,0 et 15,5 Hz, 1H) ; 7.48 (t, J = 9.0 Hz, 1H); 7,69 (ddd, J = 2,5 - 5,5 et 9,0 Hz, 1H) ; 7,81 (dd, J = 2,5 et 6,5 Hz, 1H) ; 7,91 (d, J = 8,5 Hz, 1H).

### Exemple 25 : N-[(S)-1-(3-cyno-4-fluorobenzyl)-2-oxo-perhydro-azépin-3-yl]-(E)-(2R,3R,4S,5R)-3,4,5-Trihydroxy-2-méthoxy-8-méthyl-dec-6-ènamide

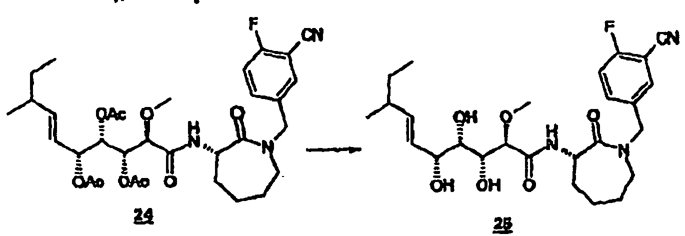

Dans un ballon de 50 ml muni d'une agitation magnétique, on introduit successivement 10,0 ml de méthanol, 267 mg (423 µmol) du composé 24 et 187 mg (1,35 mmol, 3,2 éq.) de carbonate de potassium. Le milieu réactionnel est agité 10 minutes à température ambiante, versé dans 100 ml d'une solution, saturée de NH₄Cl, puis extrait avec de l'acétate d'éthyle (2 fois 100 ml). La phase aqueuse est saturée avec NaCl, et extraite avec 100 ml d'AcOEt. Les phases organiques sont réunies et évaporées à sec, on obtient 226 mg de produit brut, qui est chromatographié sur une cartouche de silice (9g, éluant, CH₂Cl₂/Isopropanol en gradient : Isopropanol 0 à 10%) pour donner 121 mg (57%) de produit 25 attendu.

ES: 506(+)=(M+H)(+)

Spectre RMN 1H (400MHz) - δ en ppm - DMSO-d6, mélange d'isomères (80 % - 20 %) : 0,80 (t, J = 7,6 Hz, 2,4H) ; 0,82 (t, J = 7,6 Hz, 0,6H) ; 0,92 (d, J = 7,0 Hz, 0,6H) ; 0,93 (d, J = 7,0 Hz, 2,4H) ; 1,14 (m, 1H) ; de 1,20 à 1,30 (m, 2H) ; 1,40 (m, 1H); de 1,80 à 1,71 (m, 2H) ; de 1,76 à 1,94 (m, 2H) ; 1,98 (m, 1H) ; de 3,24 à 3,37 (m partiellement masqué, 2H) ; 3,27 (s, 3H) ; de 3,55 à 3,67 (m, 2H).; 3,71 (d, J = 7,5 Hz, 1H) ; 3,98 (m, 1H) ; 4,32 (d, J = 4,0 Hz, 0,2H) ; 4,39 (m large, 1H); 4,45 (m partiellement masqué, 1H) ; 4,46 (d, J = 15,0 Hz, 1H); 4,57 (m large, 0,8H) ; 4,65 (m, 1H) ; 4,69 (d, J = 15,0 Hz, 1H) ; 5,37 (m, 1H) ; 5,49 (dd, J = 7,5 et 16,6 Hz, 1H) ; 7,50 (t, J = 9,0 Hz, 1H) ; 7.68 (ddd, J = 2,5 - 5,5 et 9,0 Hz, 1H) ; 7,81 (dd, J = 2,5 et 6,5 Hz, 1H) ; 7,90 (d, J = 6,5 Hz, 1H).

### Activité antiproliférative des produits préparés:

L'activité antiproliférative du produit 3 a été déterminée par mesure de l'inhibition de ta prolifération cellulaire de cellules Hep-G2. Les cellules sont ensemencées dans un milieu de culture cellulaire à une concentration de 1000 cellules par puits, et incubées pendant 4 heures à 37°C et 5% de CO₂.

Milieu utilisé pour la culture de cellules Hep-G2 : Milieu de Eagle, modifié par Dubelcco/Mélange F12 de Ham Gibco) ; NEAA (10% ; acides aminés non essentiels, Gibco) ; pyruvate de sodium (1%, Gibco) ; L-glutamine (1%, Gibco) ; Sérum de veau foetal (5% ; PAA).

A l'issue des 4 heures, les produit à tester dissous dans un mélange DMSO/milieu de culture cellulaire, sont ajoutés à des concentrations variées et les mélanges résultants sont incubés pendant 72 heures à 37°C et 5% de CO₂. Le contenu intracellulaire en ATP a été mesure en utilisant la réactif d'essai CellTiterGlo (Promega).

Les résultats des tests de prolifération cellulaire sont donnés dans le tableau 1, ci-après:

**- Tableau 1 -**

| **Exemple** | **Structure** | **IC 50 (µM)/HEP G2** |
|---|---|---|
| Bengamide E | 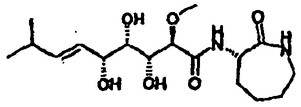 | 7.0 |
| 3 | 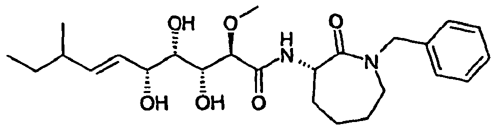 | 0.02 |

L'activité antiproliférative des produits des exemples du tableau 2 a été déterminée par mesure de l'inhibition de la prolifération cellulaire de cellules HCT116. Les cellules sont ensemencées dans un milieu de culture cellulaire à une concentration de 10 000 cellules par puits, dans 0.17 mL de milieu, et 20 µL de produit à tester, à différentes concentrations, et 10 µL de Thymidine [methyl-14C] (100 µCi/ml - activité spécifique 47.90 mCi/mmol; NEN Technologies référence NEC568 batch 3550-001) sont ajoutés, puis les cellules sont incubées à 37°C et 5% de CO₂.

Milieu utilisé pour la culture de cellules HCT116 : milieu DMEM 2 mM L-glutamine, 200 UI/ml pénicilline, 200 µg/ml streptomycine and 10% (V/V) Sérum de veau foetal (Life Technologies).

Après 48 heures, l'incorporation de ¹⁴C-thymidine est comptée dans un compteur à scintillation liquide 1450 Microbeta Wallac Trilux. Les résultats R sont exprimés en cpm (coups par minute) et convertis en pourcentage d'inhibition de croissance GI% en faisant premièrement la soustraction de la moyenne du nombre de cpm des puits sans cellules B et en divisant ensuite par le nombre de cpm des puits des cellules non traitées C comprenant 20µL de milieu de dilution du produit contentant 1 % d'éthanol. (GI % = (R - B) x 100 / C %).
Les valeurs d'IC50 sont calculées à l'aide de l'équation 205 du logiciel XLFit (IDBS company, UK) par analyse de régréssion non linéaire utilisant l'algorithme Marquardt (Donald W. MARQUARDT, J-Soc.industry.appl, vol 11, No. 2, June, 1963).

Les produits du tableau 2 présentent une IC50 sur les cellules HCT116 généralement inférieure à 30µM, et de préférence inférieure à 100nM.

**- Tableau 2 -**

| **Exemple** | **Structure** |
|---|---|
| 13 | 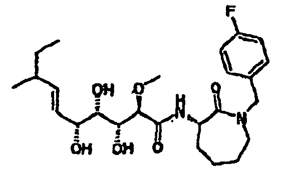 |
| 15 | 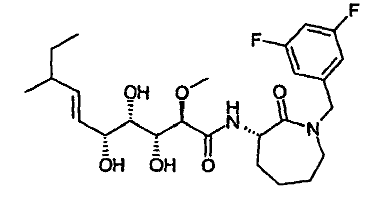 |
| 17 | 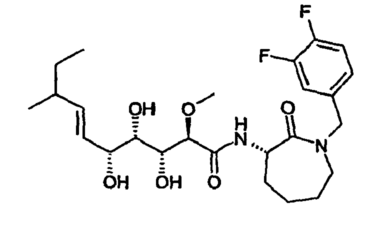 |
| 19 | 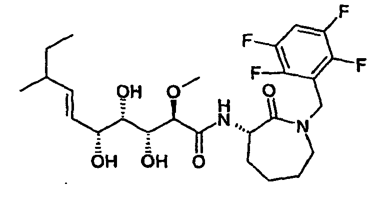 |
| 21 | 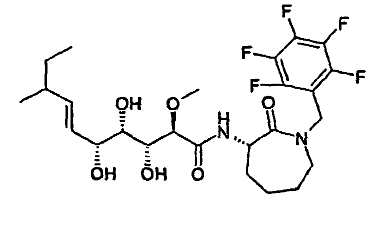 |
| 23 | 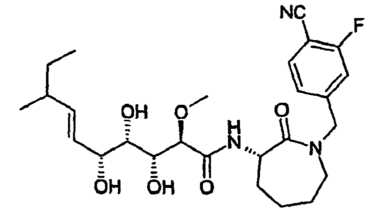 |
| 25 | 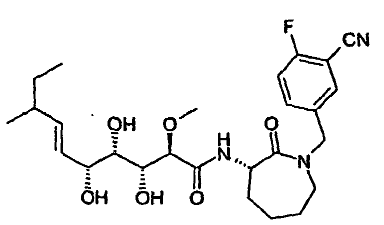 |

## Revendications

1. Produit de formule générale (II) suivante : 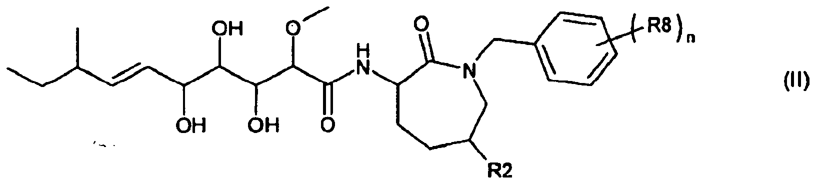 dans lequel chaque R8 est indépendamment sélectionné dans le groupe constitué par H, halogène, OH, CN, O(C1-C24)alkyle, OCO(C1-C24)alkyle, -(C1-C4)alkyl-aryle, -(C1-C4)alkyl-hétéroaryle, les groupes aryles comprenant de 6 à 14 atomes de carbone et les groupes hétéroaryles comprenant de 1 à 13 atomes de carbone et de 1 à 4 hétéroatomes ; dans lequel n = 0, 1, 2, 3, 4 ou 5, et dans lequel R2 est sélectionné dans le groupe constitué par H ou OH.

2. Produit selon la revendication 1, **caractérisé en ce que** R8 est indépendamment sélectionné dans le groupe constitué par H et F, et dans lequel n = 0, 1, 2, 3, 4 ou 5.

3. Produit selon l'une quelconque des revendications 1 ou 2, **caractérisé en ce que** n = 4 ou 5.

4. Produit selon la revendication 1, **caractérisé en ce qu'**il s'agit de :

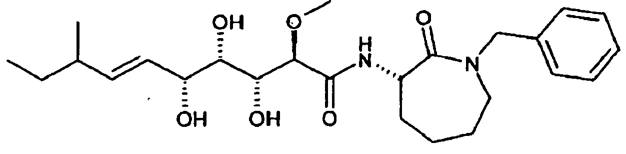

5. Produit selon l'une quelconque des revendications 1 à 4, **caractérisé en ce qu'**il s'agit de :
N-[(S)-1-benzyl-2-oxo-perhydro-azépin-3-yl]-(E)-(2R,3R,4S,5R)-3,4,5-Trihydroxy-2-méthoxy-8-méthyl-dec-6-ènamide,
N-[(S)-1-(4-fluoro-benzyl)-2-oxo-perhydro-azépin-3-yl]-(E)-(2R,3R,4S,5R)-3,4,5-Trihydroxy-2-méthoxy-8-méthyl-dec-6-ènamide,
N-[(S)-2-oxo-1-(3,5-difluorobenzyl)-perhydro-azépin-3-yl]-(E)-(2R,3R,45,5R)-3,4,5-Trihydroxy-2-méthoxy-8-méthyl-dec-6-ènamide,
N-[(S)-1-(3,4-difluoro-benzyl)-2-oxo-perhydro-azépin-3-yl]- (E)- (2R,3R,4S,5R)-3,4,5-Trihydroxy-2-méthoxy-8-méthyl-dec-6-ènamide,
N-[(S)-1-(2,3,5,6-tetrafluoro-benzyl)-2-oxo-perhydro-azépin-3-yl]-(E)-(2R,3R,4S,5R)-3,4,5-Trihydroxy-2-méthoxy-8-méthyl-dec-6-ènam ide,
N-[(S)-1-(2,3,4,5,6-pentafluorobenzyl)-2-oxo-perhydro-azépin-3-yl]-(E)-(2R,3R,4S,5R)-3,4,5-Trihydroxy-2-méthoxy-8-méthyl-dec-6-ènamide,
N-[(S)-1-(4-cyano-3-fluorobenzyl)-2-oxo-perhydro-azépin-3-yl]-(E)-(2R,3R,45,5R)-3,4,5-Trihydroxy-2-méthoxy-8-méthyl-dec-6-énamide,
N-[(S)-1-(3-cyano-4-fluorobenzyl)-2-oxo-perhydro-azépin-3-yl]-(E)-(2R,3R,4S,5R)-3,4(5-Trihydroxy-2-méthoxy-8-méthyl-dec-6-ènamide,

6. Produit selon l'une quelconque des revendications précédentes, **caractérisé en ce qu'**il est sous forme :
1) racémique, ou ,
2) enrichie en un stéréo-isomère, ou
3) enrichie en un énantiomère ;
et **en ce qu'**il est éventuellement salifié.

7. Procédé de préparation d'un produit de formule générale (II) selon la revendication 1, comprenant une étape dans laquelle un produit de formule générale (VI) suivante : 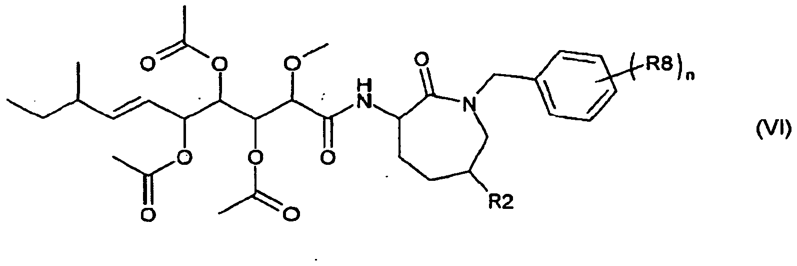 dans laquelle R2 est H ou OCOCH₃, et R8 et n sont tels que définis précédemment, est saponifié pour l'obtention d'un produit de formule générale (II).

8. Procédé selon la revendication 7, **caractérisé en ce que** le produit de formule générale (VI) est obtenu par réaction entre un produit de formule générale (V) suivante : 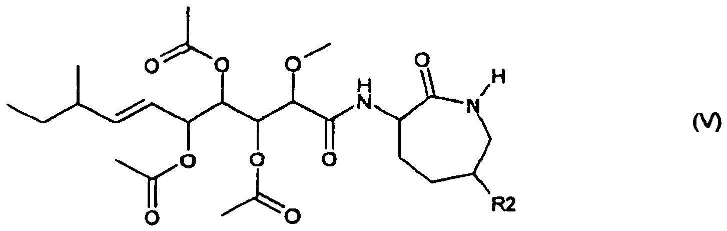 et un halogénure de benzyle 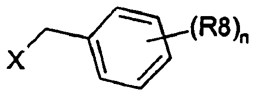 dans lequel X est un halogène et R8 et n sont tels que définis précédemment, en présence d'une base.

9. Procédé selon la revendication 8, **caractérisé en ce que** le produit de formule générale (V) est obtenu par acétylation d'un produit de formule générale (IV) suivante : 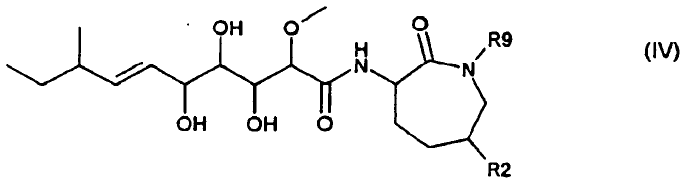 dans laquelle R9 est H, et R2 est H ou OH.

10. Composition pharmaceutique comprenant un produit selon l'une quelconque des revendications 1 à 6, en combinaison avec un excipient pharmaceutiquement acceptable.

11. Produit selon l'une quelconque des revendications 1 à 6, pour son utilisation pour le traitement d'un état pathologique.

12. Produit selon l'une quelconque des revendications 1 à 6, pour son utilisation pour le traitement du cancer.

## Claims

1. Product of general formula (II) below: 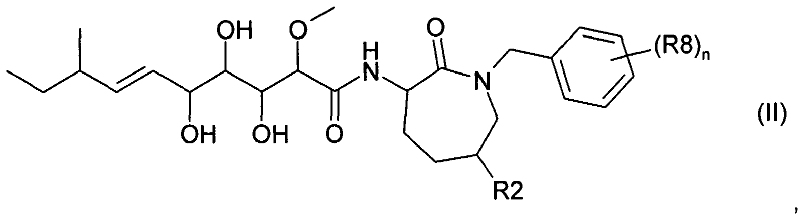 in which each R8 is independently selected from the group consisting of H, halogen, OH, CN, O(C1-C24)alkyl, OCO(C1-C24)alkyl, -(C1-C4)alkyl aryl, -(C1-C4)alkyl-heteroaryl, aryl groups comprising from 6 to 14 carbon atoms and heteroaryl groups comprising from 1 to 13 carbon atoms and from 1 to 4 heteroatoms; in which n = 0, 1, 2, 3, 4 or 5 and in which R2 is selected from the group formed by H and OH.

2. Product according to Claim 1, **characterized in that** R8 is independently selected from the group consisting of H and F, and in which n = 0, 1,2, 3, 4 or 5.

3. Product according to either one of Claims 1 and 2, **characterized in that** n = 4 or 5.

4. Product according to Claim 1, **characterized in that** it is:

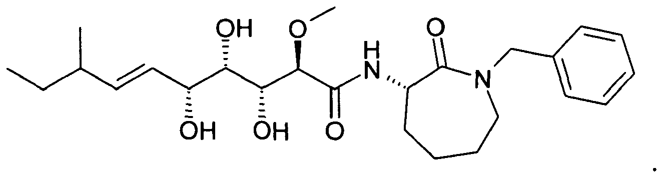

5. Product according to any one of Claims 1 to 4, **characterized in that** it is:
N-[(S)-1-benzyl-2-oxoperhydroazepin-3-yl]-(E)-(2R,3R,4S,5R)-3,4,5-trihydroxy-2-methoxy-8-methyldec-6-enamide,
N-[(S)-1-(4-fluorobenzyl)-2-oxoperhydroazepin-3-yl]-(E)-(2R,3R,4S,5R)-3,4,5-trihydroxy-2-methoxy-8-methyldec-6-enamide,
N-[(S)-2-oxo-1-(3,5-difluorobenzyl)perhydroazepin-3-yl]-(E)-(2R,3R,4S,5R)-3,4,5-trihydroxy-2-methoxy-8-methyldec-6-enamide,
N-[(S)-1-(3,4-difluorobenzyl)-2-oxoperhydroazepin-3-yl]-(E)-(2R,3R,4S,5R)-3,4,5-trihydroxy-2-methoxy-8-methyldec-6-enamide,
N-[(S)-1-(2,3,5,6-tetrafluorobenzyl)-2-oxoperhydroazepin-3-yl]-(E)-(2R,3R,4S,5R)-3,4,5-trihydroxy-2-methoxy-8-methyldec-6-enamide,
N-[(S)-1-(2,3,4,5,6-pentafluorobenzyl)-2-oxoperhydroazepin-3-yl]-(E)-(2R,3R,4S,5R)-3,4,5-trihydroxy-2-methoxy-8-methyldec-6-enamide,
N-[(S)-1-(4-cyano-3-fluorobenzyl)-2-oxoperhydroazepin-3-yl]-(E)-(2R,3R,4S,5R)-3,4,5-trihydroxy-2-methoxy-8-methyldec-6-enamide,
N-[(S)-1-(3-cyano-4-fluorobenzyl)-2-oxoperhydroazepin-3-yl]-(E)-(2R,3R,4S,5R)-3,4,5-trihydroxy-2-methoxy-8-methyldec-6-enamide.

6. Product according to any one of the preceding claims, **characterized in that** it is in:
1) racemic form, or
2) a form enriched in one stereoisomer, or
3) a form enriched in one enantiomer;
and **in that** it is optionally salified.

7. Process for preparing a product of general formula (II) according to Claim 1,
comprising a step in which a product of general formula (VI) below: 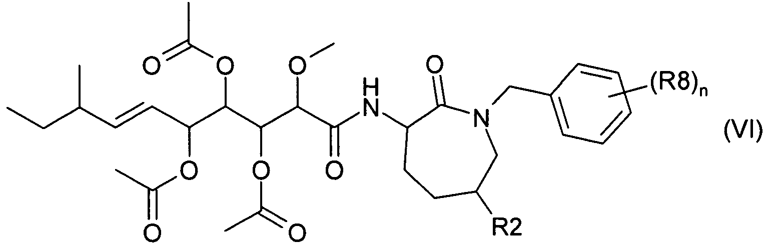 in which R2 is H or OCOCH₃, and R8 and n are as defined above, is saponified to obtain a product of general formula (II).

8. Process according to Claim 7, **characterized in that** the product of general formula (VI) is obtained by reaction between a product of general formula (V) below: 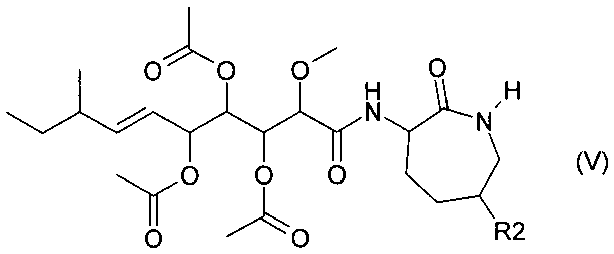 and a benzyl halide 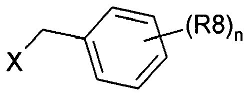 in which X is a halogen and R8 and n are as defined above, in the presence of a base.

9. Process according to Claim 8, **characterized in that** the product of general formula (V) is obtained by acetylation of a product of general formula (IV) below: 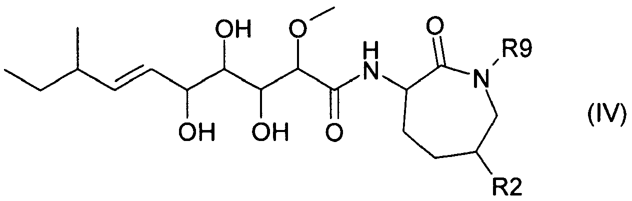 in which R9 is H and R2 is H or OH.

10. Pharmaceutical composition comprising a product according to any one of Claims 1 to 6, in combination with a pharmaceutically acceptable excipient.

11. Product according to any one of Claims 1 to 6, for the use thereof for the treatment of a pathological condition.

12. Product according to any one of Claims 1 to 6, for the use thereof for the treatment of cancer.

## Patentansprüche

1. Produkt der folgenden allgemeinen Formel (II): 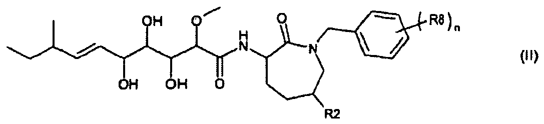 in der R8 jeweils unabhängig aus der Gruppe bestehend aus H, Halogen, OH, CN, O(C1-C24)-Alkyl, OCO(C1-C24)-Alkyl, -(C1-C4)-Alkylaryl, -(C1-C4)-Alkylheteroaryl ausgewählt ist, wobei die Arylgruppen 6 bis 14 Kohlenstoffatome umfassen und die Heteroarylgruppen 1 bis 13 Kohlenstoffatome und 1 bis 4 Heteroatome umfassen; worin n = 0, 1, 2, 3, 4 oder 5 und in der R2 aus der Gruppe bestehend aus H oder OH ausgewählt ist.

2. Produkt nach Anspruch 1, **dadurch gekennzeichnet, dass** R8 unabhängig aus der Gruppe bestehend aus H und F ausgewählt ist und worin n = 0, 1, 2, 3, 4 oder 5.

3. Produkt nach einem der Ansprüche 1 oder 2, **dadurch gekennzeichnet, dass** n = 4 oder 5.

4. Produkt nach Anspruch 1, **dadurch gekennzeichnet, dass** es sich um: 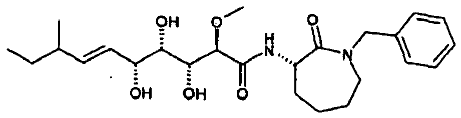 handelt.

5. Produkt nach einem der Ansprüche 1 bis 4, **dadurch gekennzeichnet, dass** es sich um
N-[(S)-1-Benzyl-2-oxoperhydroazepin-3-yl]-(E)-(2R,3R,4S,5R)-3,4,5-trihydroxy-2-methoxy-8-methyldec-6-enamid,
N-[(S)-1-(4-Fluorbenzyl)-2-oxoperhydroazepin-3-yl]-(E)-(2R,3R,4S,5R)-3,4,5-trihydroxy-2-methoxy-8-methyldec-6-enamid,
N-[(S)-2-oxo-1-(3,5-Difluorbenzyl)perhydroazepin-3-yl]-(E)-(2R,3R,4S,5R)-3,4,5-trihydroxy-2-methoxy-8-methyldec-6-enamid,
N-[(S)-1-(3,4-Difluorbenzyl)-2-oxoperhydroazepin-3-yl]-(E)-(2R,3R,4S,5R)-3,4,5-trihydroxy-2-methoxy-8-methyldec-6-enamid,
N-[(S)-1-(2,3,5,6-Tetrafluorbenzyl)-2-oxoperhydroazepin-3-yl]-(E)-(2R,3R,4S,5R)-3,4,5-trihydroxy-2-methoxy-8-methyldec-6-enamid,
N-[(S)-1-(2,3,4,5,6-Pentafluorbenzyl)-2-oxoperhydroazepin-3-yl]-(E)-(2R,3R,4S,5R)-3,4,5-trihydroxy-2-methoxy-8-methyldec-6-enamid,
N-[(S)-1-(4-Cyano-3-fluorbenzyl)-2-oxoperhydroazepin-3-yl]-(E)-(2R,3R,4S,5R)-3,4,5-trihydroxy-2-methoxy-8-methyldec-6-enamid,
N-[(S)-1-(3-Cyano-4-fluorbenzyl)-2-oxoperhydroazepin-3-yl]-(E)-(2R,3R,4S,5R)-3,4,5-trihydroxy-2-methoxy-8-methyldec-6-enamid
handelt.

6. Produkt nach einem der vorhergehenden Ansprüche, **dadurch gekennzeichnet, dass** es in
1) racemischer Form oder
2) in stereoisomerenangereicherter Form oder
3) in enantiomerenangereicherter Form
vorliegt und dass es gegebenenfalls als Salz vorliegt.

7. Verfahren zur Herstellung eines Produkts der allgemeinen Formel (II) nach Anspruch 1, das einen Schritt umfasst, in dem man ein Produkt der folgenden allgemeinen Formel (VI): 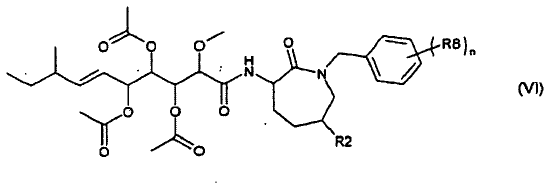 in der R2 H oder OCOCH₃ bedeutet und R8 und n wie zuvor definiert sind, verseift, wodurch man zu einem Produkt der allgemeinen Formel (II) gelangt.

8. Verfahren nach Anspruch 7, **dadurch gekennzeichnet, dass** man das Produkt der allgemeinen Formel (VI) dadurch erhält, dass man ein Produkt der folgenden allgemeinen Formel (V): 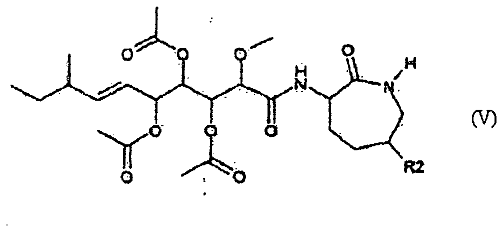 und ein Benzylhalogenid 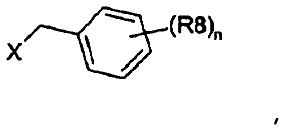 in dem X Halogen bedeutet und R8 und n wie zuvor definiert sind, in Gegenwart einer Base umsetzt.

9. Verfahren nach Anspruch 8, **dadurch gekennzeichnet, dass** man das Produkt der allgemeinen Formel (V) dadurch erhält, dass man ein Produkt der folgenden allgemeinen Formel (IV) : 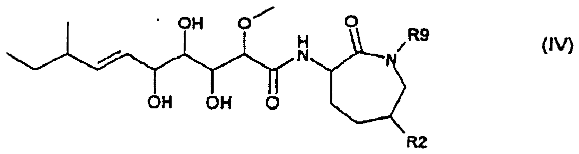 in der R9 H bedeutet und R2 H oder OH bedeutet, acetyliert.

10. Pharmazeutische Zusammensetzung, umfassend ein Produkt nach einem der Ansprüche 1 bis 6 in Kombination mit einem pharmazeutisch unbedenklichen Grundstoff.

11. Produkt nach einem der Ansprüche 1 bis 6 für die Verwendung für die Behandlung eines pathologischen Zustands.

12. Produkt nach einem der Ansprüche 1 bis 6 für die Verwendung für die Behandlung von Krebs.
